(19) Europäisches Patentamt
European Patent Office
Office européen des brevets

(11) Publication number: **0 102 425**
**B1**

(12) # EUROPEAN PATENT SPECIFICATION

(45) Date of publication of patent specification: **09.04.86**

(51) Int. Cl.$^4$: **C 07 C 149/00,**
C 10 M 105/72, C 10 L 1/24

(21) Application number: **82304645.3**

(22) Date of filing: **03.09.82**

(54) **Triglyceride-based additive for oils and method of preparing the additive.**

(43) Date of publication of application:
**14.03.84 Bulletin 84/11**

(45) Publication of the grant of the patent:
**09.04.86 Bulletin 86/15**

(84) Designated Contracting States:
**DE FR GB**

(56) References cited:
**US-A-4 149 982**
**US-A-4 166 796**

(73) Proprietor: **FERRO CORPORATION**
**One Erieview Plaza**
**Cleveland Ohio 44114 (US)**

(72) Inventor: **Kammann, Karl P., Jr.**
**12431 Van Buren Street**
**Crown Point Indiana 46307 (US)**
Inventor: **Den Herder, Marvin J.**
**816 Brookwood Drive**
**Olympia Fields Illinois 60461 (US)**

(74) Representative: **Wain, Christopher Paul et al**
**A.A. THORNTON & CO. Northumberland House**
**303-306 High Holborn**
**London WC1V 7LE (GB)**

**EP 0 102 425 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

It has been common practice to include in lubricant formulations additives to provide improved antiwear and rust inhibition properties. In the past, sulfurized triglycerides, such as sulfurized lard oil, have been utilized, especially in association with lightly refined aromatic mineral oils which provided sufficient solubility for the sulfurized triglycerides.

With the increased concern for toxicity of aromatic compounds found in such mineral oils, lubricant formulations now comprise essentially non-aromatic oils. This change to substantially non-aromatic base oils created a major problem, resulting from a significant decrease in solubility of the sulfurized triglycerides in the non-aromatic mineral oil, resulting in solidification and/or dropout of the sulfurized triglycerides.

While the solubility problem has been overcome, the modified lubricant products have been found to be either deficient in desirable lubricant properties or incapable of providing needed improvement in these properties.

In a typical approach to this problem, as reported in U.S. Patent No. 3,455,896, sulfurized, low molecular weight polybutenes were reacted with liquid triglycerides, which were susceptible of sulfurization, to yield an additive. In U.S. Patent No. 3,850,825, another additive was prepared by the sulfurization of a mixture of prime burning lard oil and alkyl oleate. In U.S. Patent No. 3,740,333, $C_{10}$—$C_{16}$ alcohol esters of unsaturated fatty acids, having 18 to 22 carbon atoms, were blended with a triglyceride and either used "as is" or sulfurized. Modifications of such compositions have been reported in U.S. Patent Nos. 4,149,982, 4,166,795, 4,166,796, 4,166,797, and 4,188,300.

Although these prior art efforts have increased the solubility of sulfurized fatty oils to acceptable values, there has remained a serious need for sulfurized additives possessing both good solubility and a combination of improved lubricant properties, such as, for example, better low temperature flow properties, better load carrying and antifriction properties, and a lack of sludging. Such improved lubricant properties would also be attractive for use in various fuels systems employed for power generation and heating purposes.

Summary of the invention

This invention relates to improved lubricant additive compositions comprising sulfurized fatty oils, to the process for their preparation, and to oil product compositions, including both fuels and lubricants, incorporating such sulfurized fatty oils. The additive compositions of this invention exhibit highly desirable solubility properties when employed in either lubricant or fuel formulations. The particularly desirable utility of these additive compositions derives from their providing generally improved performance characteristics, ranging from improved load carrying, antiwear, and friction properties, to reduced levels of deposits and varnish, and to improved pour-point depression.

This invention particularly relates to sulfurized, fatty oil additive compositions, comprising a sulfurized, transesterified triglyceride wherein the total acid component of the triglyceride comprises no less than about 35 mole % saturated aliphatic acids and no more than about 65 mole % unsaturated fatty acids, said total acid component being further characterized as comprising:

a) more than about 20 mole % of mono-unsaturated acids;

b) less than about 15 mole % of poly-unsaturated fatty acids;

c) more than about 20 mole % saturated aliphatic acids having 6 to 16 carbon atoms, including more than about 10 mole % saturated aliphatic acids having 6 to 14 carbon atoms; and

d) less than about 15 mole % saturated aliphatic acids having 18 or more carbon atoms.

This invention further relates to the method for preparation of such transesterified and sulfurized triglycerides.

This invention additionally relates to lubricant and fuel compositions incorporating such sulfurized, fatty oil additives, whereby improved performance in conventional usages is achieved. The additives of this invention may be employed in concentrations up to about 15 wt. % in lubricant formulations and up to about 0.1 wt. % in fuel compositions.

Description of the invention

This invention is directed to additive compositions of sulfurized fatty oils, and to the process of preparing said compositions, which exhibit the required solubility properties in non-aromatic base oils without the disadvantages associated with the prior art lubricant additive formulations. In addition, the compositions of this invention, exhibit improved performance characteristics, over the compositions of the prior art, including improved load carrying, antiwear, and friction properties, reduced levels of deposits and varnish in used oils, and better pour-point depression. This invention is likewise directed to lubricant and fuel formulations which include the inventive additive compositions.

Triglycerides of the prior art, typically derived from plants and animals, do not provide maximum effectiveness as lubricant additives because of the chain length and/or the degree of unsaturation of the acid moiety. Modification of said acid moieties of the triglycerides, by transesterification, produces novel

triglycerides that optimize the properties of the resulting additive when said novel triglycerides are coupled, through sulfur bonds, with solubilizing components, such as esters and/or olefins.

The acid moiety of the triglyceride components of the additives of this invention consists of an acid mixture having less than about 65 mole % unsaturated acids, mainly possessing one ethylenic carbon-carbon double bond, and more than 35 mole % saturated aliphatic acids. Of the total acid moiety, less than about 15 mole % are saturated acids having 18 or more carbon atoms and more than about 35 mole % are saturated acids having less than 18 carbon atoms. Similarly, less than about 15 mole % are poly-unsaturated acids and more than about 20 mole % are mono-unsaturated acids.

The transesterification reaction is carried out on blends of (1) triglycerides, (2) triglycerides and organic acids, or (3) triglycerides and esters of organic acids. Where acids are included in the reaction and the amount of free acids present in the transesterified blend is greater than about 15%, then the free acid is preferably esterified with monoalcohols, glycols or glycerol to decrease the free acid content prior to the subsequent sulfurization reaction. The esterification of free acids may also be effected when the concentration thereof is less than about 15% but this is not imperative.

Following the transesterification, or esterification, the reaction components are coupled by reaction with sulfur with, where desired, the added presence of solubilizing components, such as esters, olefins or blends thereof. The sulfurization is conducted in accordance with known procedures which generally consist of heating the mixture with elemental sulfur at temperatures from about 300°F. to about 400°F. (149 to 204°C) for about 1 to about 8 hours. The sulfur content of the additives of this invention should preferably be within the range from about 4 to about 14 wt.%.

The additives of the present invention preferably utilize as starting compounds naturally occurring triglycerides. The compositions of such triglycerides are detailed in *Bailey's Industrial Oil and Fat Products*, Vol. I, 4th Edition, John Wiley and Sons.

A triglyceride is the ester product of glycerol and one or more fatty acids, represented schematically as

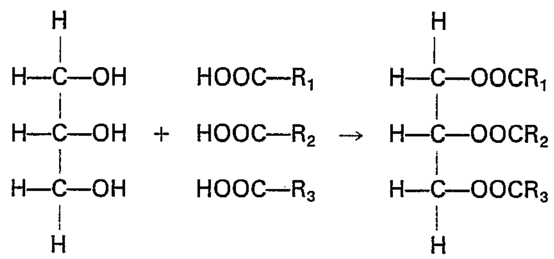

where $R_1$, $R_2$, and $R_3$ represent hydrocarbon groups which may be identical or different in chain length and may also be saturated or unsaturated.

Triglycerides from fish and animal oils contain acids with chain lengths that normally exceed 15 carbon atoms and usually contain large amounts of mono- and poly-unsaturated acids. Triglycerides from some plant species contain appreciable amounts of shorter chain acids, having 10, 12 or 14 carbon atoms. These shorter chain plant-derived acids tend to be mainly saturated acids.

In the process of preparing the triglyceride components of the additive of the present invention, commercially available triglycerides which do not have the required distribution of acids are transesterified with acids, esters or triglycerides having a higher proportion of the required distribution of acids. The resultant mixtures, following the transesterification, have the required average distribution of acids, preferably as triglycerides. Free acids in the transesterification reaction product may then be esterified with mono-alcohols, ranging from methyl to $C_{20}$, or with poly-alcohols, such as a glycol or glycerol. If the free acid content in the transesterification reaction product is greater than about 15%, esterification is a highly preferred procedure, whereas below the 15% level esterification is optional. Transesterification is preferably carried out in the presence of a strong acid catalyst, at temperatures within the range from about 400°F. to about 450°F. (204 to 232°C) for from about 1 to about 8 hours.

Prior to sulfurization, the transesterified mixture may be blended with a solubilizing component when further improved solubility is desired. Whenever there is a sufficient amount of free unsaturated acid in the transesterification reaction product, the esterification of such acids can provide the solubilizing factor. Otherwise additional solubilizing components, such as unsaturated esters or olefins are added prior to sulfurization.

Although the amount of solubilization component present, prior to sulfurization may, if desired, be as high as about 70 wt. %, such solubilization components, when employed, are preferably present in an amount within the range from about 5 wt. % to about 55 wt. %.

Examples of naturally occurring triglycerides, which may be utilized as starting triglycerides for the preparation of the additives of this invention, include, but are not limited to, lard oil, tallow, palm oil and peanut oil.

The acid moiety of the triglyceride, following transesterification, consists of a total mono- and poly-unsaturated fatty acids in an amount of less than about 65 mole %. The acid moiety consists of more than about 20 mole %, preferably more than about 35 mole %, of mono-unsaturated fatty acids and less

than about 15 mole % poly-unsaturated fatty acids; i.e., acids having more than one ethylenic carbon-carbon bond. Total saturated aliphatic acids comprise more than about 35 mole %, and preferably more than about 50 mole %, of said acid moiety. Of the total acid moiety, saturated acids having 6 to 14 carbon atoms are present in an amount of more than about 10 mole % and preferably more than about 15 mole %; saturated acids having 6 to 16 carbon atoms including the aforementioned acids having 6 to 14 carbon atoms, are present in an amount of more than about 20 mole % and preferably more than about 35 mole %; and saturated acids having 18 or more carbon atoms are present in an amount of less than about 15 mole %.

The acids utilized are normally straight chain acids, although the presence of some branched chain acids, such as 2-ethylhexanoic and 2-methyldecanoic, is not deleterious.

Almost any alcohol can be utilized in the optional esterification step, including glycerol, diols and monohydroxy alcohols, especially terminal primary alcohols. Branched alcohols, such as 2-ethylhexyl, isodecyl, isododecyl and mixtures containing a wide range of alcohols, such as 11 to 22 carbon atoms, can also be utilized.

Olefins, when used as solubilizing agents, normally contain 8 to 20 carbon atoms per molecule. Olefin mixtures may be employed.

In one embodiment of this invention, the transesterification and esterification is caused to occur in a reduced number of steps by mixing together the starting triglyceride, replacement acids, and alcohol, and then subjecting the mixture to heat in the presence of a transesterification catalyst.

Transesterification catalysts are normally utilized to speed the reaction, although the reaction will proceed without a catalyst. The amount and type of catalyst can be widely varied. Known transesterification catalysts are tetrabutyl titanate, zinc acetate, sodium carbonate, sodium hydroxides, potassium hydroxide, sodium methylate, sodium sulfate, stannous oxalate, p-toluenesulfonic acid (PTSA), methanesulfonic acid (MSA), butylchlorotin dihydroxide, sulfuric acid, phosphoric acid and the like. p-Toluenesulfonic acid and methanesulfonic acid are the preferred catalyst. The amount of catalyst utilized is in the range of about 0.01 wt. % to about 1 wt. % with the preferred range being about 0.03 wt. % to 0.5 wt. %.

Comparison runs made with and without a transesterification catalyst showed the following degree of completion:

a) catalyst: 0.15% methanesulfonic acid, 4 hours at 400°F. (204°C)—about 72% completion;
b) catalyst: 0.1% butylchlorotin dihydroxide, 8 hours at 400°F. (204°C)—about 44% completion; and
c) catalyst: NONE—8 hours at 400°F. (204°C)—about 21% completion.

The above comparison study shows that the transesterification reaction occurs even in the absence of a catalyst and that the rate of reaction is increased by the addition of a catalyst.

During the transesterification reaction the presence of 0.15 to 1.0 wt. % water increases the rate of transesterification. However, during subsequent esterification it is desirable that the water that had been added, or is generated by the esterification reaction, be removed as promptly as possible, in order to drive the reaction to completion and thus increase the yield.

The following examples serve, without limitation, to describe the invention more fully as it relates to lubricant additive compositions. In the examples all parts and percentages are on a weight basis unless otherwise indicated.

In the following examples HOE alcohol refers to an 11 to 22 carbon alkyl alcohol, averaging about 16 carbons, mainly branched primary alcohol, sold commercially as Heavy Oxo Ends. Emery 876 acid is a saturated acid mixture containing about 11% $C_9$, 2% $C_{10}$—$C_{13}$, 16% $C_{14}$, 1% $C_{15}$, 42% $C_{16}$, 1% $C_{17}$ and 12% $C_{18}$ monobasic acids and 15% $C_6$—$C_{14}$ dibasic acids. Diol concentrate is a mixture of predominantly straight chain alcohols, containing about 84% diols, mainly $C_{13}$—$C_{17}$ primary, and about 16% monohydroxy alcohols, mainly $C_{15}$—$C_{16}$ primary.

Example 1

A blend of 67 parts prime lard oil, 28 parts crude coconut oil, and 5 parts oleic acid was heated for 4 hours at 400 to 410°F. (204 to 210°C) in the presence of 0.2% p-toluenesulfonic acids. The acid value (A.V.) of the mixture rose from 16 to 20. Acid value is determined by titration (A.O.C.S. method Cd 3a-63) and is defined as the number of milligrams of potassium hydroxide necessary to neutralize the free acids in one gram of sample.

To the transesterification reaction mixture I was added 8 parts of HOE alcohol and the heating continued for an additional 3 hours. The A.V. value was reduced during this process to 10. The resultant product II, except for small amounts of free acid and alcohol, contained about 87% transesterified triglyceride and about 13% ester.

This product was sulfurized by heating with elemental sulfur at 360—370°F. (182—188°C) for 3 hours, followed by cooling below 330°F. (166°C) and passing air through the mixture for about 1.5 hours, to remove any $H_2S$ or other noxious light ends. The resultant product III contained 6.3% bound sulfur.

Example 2

Sixty (60%) percent of the transesterified triglyceride product I, obtained by the procedure of Example

1, was blended with 40% of an alkyl alcohol (HOE) ester of unsaturated fatty acids (tall oil fatty acids) and the mixture sulfurized to yield product IV, having 7.3% bound sulfur.

Example 3

A blend of 100 parts prime lard oil and 25 parts Emery 621 coconut fatty acid was heated for 4 hours at 400—410°F. (204—210°C), in the presence of 0.2% p-toluenesulfonic acid. To this mixture was added 25 parts HOE alcohol and the mixture was heated for an additional 3 hours. The A.V. of this product V was 11.

To 150 parts of reaction product V was added 20 parts of the alkyl alcohol (HOE) ester of unsaturated fatty acids (tall oil fatty acids) and the mixture sulfurized to yield product VI, having 6.7% bound sulfur.

Example 4

A blend of 78 parts of a solid triglyceride, having a melting point of about 100°F. (38°C), and 22 parts Emery 621 coconut fatty acids was heated at 400—410°F. (204—210°C) for 4 hours, in the presence of 0.2% methanesulfonic acid. Twenty (20) parts of HOE alcohol was added and the reaction continued until the A.V. decreased to 10. Twenty-eight (28) parts of HOE alcohol ester of tall oil fatty acids was then added and the mixture sulfurized to yield product VII, having 6.5% bound sulfur. The mixture, prior to sulfurization, contained about 53% transesterified triglyceride and about 47% HOE ester.

Example 5

Sixty-eight (68) parts of a solid triglyceride, having a melting point of about 100°F. (380°C), was mixed with 19 parts Emery 621 coconut fatty acid and 13 parts tall oil fatty acids. The mixture was heated at 400—410°F. (204—210°C) for 4 hours, in the presence of 0.15% methanesulfonic acid. Thirty (30) parts HOE alcohol was then added and the heating continued until an A.V. of 9 was obtained. The product was then sulfurized, using 6.5% sulfur, to yield the sulfurized product VIII.

Example 6

A mixture of 80 parts prime burning lard oil and 20 parts Emery 876 acids was heated for 4 hours at 400—410°F. (204—210°C), in the presence of 0.3% p-toluenesulfonic acid. Twenty-two (22) parts HOE alcohol was then added and the heating continued until an A.V. of 10 was obtained. To this mixture was added 30 parts of a diester prepared from 2 moles of tall oil fatty acids and 1 mole diol concentrate. The resultant mixture was sulfurized to give product IX, containing 7.4% bound sulfur.

Example 7

A blend of 12% Chevron $C_{15}$—$C_{18}$ α-olefin and 88% of product V was sulfurized to give product X, containing 7.1% sulfur.

Example 8

The procedure of Example 4 was repeated on a large scale, utilizing 6% sulfur in the sulfurization step. The product XI contained 5.9% bound sulfur.

Example 9

A blend of 78 parts of a solid triglyceride, having a melting point of about 100°F. (38°C), and 22 parts Emery 621 coconut fatty acids was heated at 400—410°F. (204—210°C) for 4 hours, in the presence of 0.15% methanesulfonic acid. Twenty-three (23) parts of isodecyl alcohol was then added and the heating continued for an additional 4 hours at 340—380°F (171—193°C). The A.V. was reduced during this process to 8. Sixteen (16) parts of isodecyl ester of tall oil fatty acids was then added and the mixture sulfurized, by heating with sulfur, using the procedure of Example 1, to yield product XII, containing 6.7% bound sulfur. The mixture prior to sulfurization, except for small amounts of free acid and alcohol, contained about 56% transesterified triglyceride and 44% ester.

The following products were prepared for comparison purposes.

Example A

A mixture of 88% prime burning lard oil and 12% methyl oleate was sulfurized to produce product A, containing 9.7% bound sulfur.

Example B

A mixture of 55% prime burning lard oil and 45% HOE alcohol ester of tall oil fatty acids was sulfurized to yield sulfurized product B, containing 9.0% bound sulfur and having an A.V. of 9.

Example C

A mixture of 50% prime burning lard oil and 50% isodecyl alcohol ester of tall oil fatty acids was sulfurized to yield product C, containing 9.1% bound sulfur and having an A.V. of 8.

Products exemplary of the sulfurized fatty oil additive compositions of this invention, prepared as described in Examples 1—9, above, together with comparison products A and B, were tested by conventional procedures at various concentration levels, ranging from 1 to 4 wt.%, in a mineral oil and in

three commercially available engine oils, to determine the respective effects on flow properties. Results are presented in Tables I, II, III, and IV.

The mineral oil contained no pour depressant additive and did not flow at temperatures below 0°F. (−18°C). The engine oils contained pour depressants and still flowed at −20°F (−29°C). Solubility of the products of this invention in these oils was good.

Table I shows clearly that the additives of this invention have excellent properties as pour depressants, keeping the oil fluid at lower temperatures when added to a mineral oil having a pour point of 0°F. (−18°C). However, when large amounts of the additives are added, the ability to cause flow at low temperatures is reduced.

Tables II, III and IV show that sulfurized fatty oils (Products A and B) diminish the low temperature flow properties of pour depressed engine oils. However, additives of the present invention can be used at higher concentrations without any harmful effect upon the flow properties of the same engine oils.

The improved load carrying and friction reduction properties imparted by the use of the additives of the present invention are illustrated by the data in Tables V and VI, showing the improved load carrying and friction reduction (torque) as measured by the Falex step-up test. Tests presented in Table V were conducted with a pour-depressed engine oil. Tests presented in Table VI illustrate the additive performance with non-formulated base oils, including a mineral oil and a synthetic lubricating oil base stock.

Falex procedures for evaluating lubricants are described in Lubrication Engineering, *24*, No. 8, 349—358 (1968). The procedure employed in these tests was as follows:

After a 5 minute warmup at 250 lbs. (114 kg), the load is increased in 250 lb. (114 kg) increments and held at each increment for one minute, until failure, which is of the weld type. Torque comparisons were also made to show differences in friction.

Crankcase oil, formulated to be a high quality SE Grade 10W40 crankcase oil, was evaluated using a four-ball machine in testing for friction and wear as described in the ASTM-D-2266 procedure. The crankcase oil alone was compared with crankcase oil containing 2% additive B or 2% additive XI. Tests were conducted at 1800 R.P.M., using a 40 kg, load, for one hour at 350°F. (177°C). The results obtained were as follows:

| Additives | Wear-scar diameter |
|---|---|
| Crankcase oil | 0.86 |
| +2% Additive B | 0.80 mm |
| +2% Additive XI | 0.57 mm |

Several products were tested for solubility in synthetic hydrocarbon oils by dissolving in Gulf Synlube 4cs with warming and stirring. The solutions were then kept at 45°F. (70°C) for 4 days and finally observed after warming to room temperature. The observed results were:

| | |
|---|---|
| 2% A | Heavy bottom layer |
| 2% B | Slight dropout |
| 2% C | Slight dropout |
| 2% VI | Very slight dropout |
| 2% VIII | Tr. Haze |
| 2% VII | Hazy |
| 2% XI | Tr. Haze |

The sulfurized fatty oil additive compositions of this invention are effective when employed in lubricating oils at concentrations ranging from about 0.05 to about 15 wt. %. The preferred concentration range is generally from about 0.5 to about 5 wt. %.

In other embodiments of this invention the sulfurized fatty oil additive compositions are effective in various types of fuels, particularly to improve the lubrication of fuel pumps; to reduce wear on pistons, rings, and cylinders; and to reduce deposit formation. Such fuels broadly include gasolines, for use in spark-ignition internal combustion engines; diesel oils, for use in compression-ignition internal combustion engines; and heating (or furnace) oils, for use inoil-fired burner assemblies. Other advantages include, when employed in fuel oils or diesel fuels, reduction of pour points and attendant reduction in plugging of oil filters. In such novel and improved fuel compositions, the additives of this invention are

effective at relatively low concentrations within the range from about 0.0005 to about 0.1 wt. %, and preferably from about 0.0015 to about 0.05 wt. %.

TABLE I

Low temperature flow of a mineral oil[1] (viscosity 27 cst. at 40°C.)

| | After 16 hours at −18°F. (−28°C) | | | |
|---|---|---|---|---|
| Additive | 1% | 2% | 3% | 4% |
| A | Flows | No flow | — | — |
| B | — | Flows | No flow | — |
| C | — | Flows | No flow | — |
| VI | — | Flows | No flow | — |
| VIII | — | Flows | No flow | — |
| XI | — | Flows | Flows | No flow |
| XII | — | Flows | Flows | No flow |

[1]Without additives, no flow at 0°F. (−18°C)

TABLE II

Commercial 10W40 oil "brand A"[1]

| | After 16 hours at −20°F. (−29°C) | | | |
|---|---|---|---|---|
| Additive | 1% | 2% | 3% | 4% |
| A | Flows | No flow | — | — |
| B | — | Flows[2] | No flow | — |
| C | — | Flows[2] | No flow | — |
| III | — | Flows | No flow | — |
| IV | — | Flows | Flows | Flows |
| VI | — | Flows | Flows | Flows[2] |
| VII | — | Flows | Flows | Flows[2] |
| VIII | — | Flows | Flows | No flow |
| IX | — | Flows | Flows | No flow |
| X | — | Flows | Flows | No flow |
| XI | — | Flows | Flows | Flows[2] |
| XII | — | Flows | Flows | Flows |

[1]Without additives, flows at −20°F (−29°C)
[2]Marginal Flow.

TABLE III

Commercial 5W30 oil "brand B"[1]

| Additive | After 16 hours at −22°F. (−30°C) 1% | 2% | 3% | 4% |
|---|---|---|---|---|
| A | Flows | No flow | — | — |
| B | Flows | Flows | No flow | — |
| C | Flows | Flows | No flow | — |
| III | — | Flows | Flows | Flows |
| IV | — | Flows | Flows | Flows |
| VI | — | Flows | Flows | No flow |
| VII | — | Flows | Flows | Flows[2] |
| VIII | — | Flows | Flows | Flows[2] |
| IX | — | Flows | Flows | No flow |

[1]Without additives, flows at −22°F. (−30°C).
[2]Marginal Flow.

TABLE IV

Commercial 10W40 oil "brand C"[1]

| Additive | After 16 hours at −20°F. (−29°C) 1% | 2% | 3% | 4% |
|---|---|---|---|---|
| A | Flows | No flow | — | — |
| B | — | Flows | Flows | Flows |
| III | — | — | Flows | Flows |
| VI | — | Flows | Flows | Flows |
| VII | — | — | Flows | Flows |

[1]Without additives, flows at −20°F. (−29°C)

TABLE V
Falex step-up test, 10W40 oil, "brand A"

| | Load before failure | | Torque at 1500 lbs (682 kg) | |
|---|---|---|---|---|
| | kg | lbs | lbs-in | kg-cm |
| Oil alone | 568 | 1250 | (45 at 1250) | (52 at 568) |
| 2% B | 682 | 1500 | 30 | 35 |
| 2% C | 682 | 1500 | 30 | 35 |
| 2% III | 795 | 1750 | 25 | 29 |
| 3% IV | 909 | 2000 | 24 | 28 |
| 2% VII | 795 | 1750 | 26 | 30 |
| 3% VII | 795—909 | 1750—2000 | 25 | 29 |
| 3% VIII | 909 | 2000 | 23 | 26 |
| 2% IX | 795—909 | 1750—2000 | 27 | 31 |
| 3% IX | 909 | 2000 | 27 | 31 |
| 3% X | 795 | 1750 | 28 | 32 |
| 2% XI | 795 | 1750 | 25 | 29 |
| 3% XI | 1023 | 2250 | 24 | 28 |
| 2% XII | 682—795 | 1500—1750 | 27 | 31 |
| 3% XII | 795 | 1750 | 26 | 30 |
| 4% XII | 909 | 2000 | 25 | 29 |

TABLE VI

Falex step-up test in non-formulated base hydrocarbons

| | lbs. (kg) Load before failure | Torque at 1250 lbs (568 kg) | |
|---|---|---|---|
| | | lbs-in | kg-cm |
| Mid-continent oil | 750 (341) | — | — |
| Oil+2% VI | 1250—1500 (568—682) | 24 | 28 |
| Gulf synfluid 4cs | 250—500 (114—227) | — | — |
| Gulf synfluid 4cs+2% VIII | 1250—1500 (568—682) | 19 | 22 |

**Claims**

1. A sulfurized, triglyceride additive composition, comprising a sulfurized, transesterified triglyceride wherein the total acid component of the triglyceride comprises no less than about 35 mole %, preferably greater than 50 mole %, saturated aliphatic acids and no more than about 65 mole % saturated fatty acids, said total acid component being further characterized as comprising:

a) more than about 20 mole %, preferably more than 35 mole %, of mono-unsaturated acids;

b) less than about 15 mole %, preferably less than 10 mole %, of poly-unsaturated fatty acids;

c) more than about 20 mole % saturated aliphatic acids having 6 to 16 carbon atoms, including more than about 10 mole % saturated aliphatic acids having 6 to 14 carbon atoms; and

d) less than about 15 mole % saturated aliphatic acids having 18 or more carbon atoms.

2. A composition according to claim 1, wherein said saturated acids having 6 to 14 carbon atoms are present in an amount greater than about 15 mole % preferably greater than about 35 mole %.

3. A composition according to claim 1 or 2, which additionally comprises a solubilization agent.

4. A composition according to claim 3, wherein the solubilization agent is a triglyceride, an olefin preferably containing from 8 to 20 carbon atoms, an ester of an unsaturated carboxylic acid, or any mixture of two or more thereof.

5. A composition according to claim 3 or 4, wherein the solubilization agent is present in an amount within the range from about 5 wt. % to about 70 wt. %, preferably 5 wt. % to about 55 wt. %1 based on the transesterified triglyceride.

6. A composition according to any of claims 1 to 5, wherein bound sulfur is present in an amount within the range from about 4 wt. % to about 14 wt. %, based on the total product.

7. A method for preparing a sulfurized, transesterified triglyceride additive composition, as claimed in claim 6, comprising the steps of:

(1) transesterifying one or more triglycerides with one or more organic acids, or esters thereof, to yield a transesterified triglyceride wherein the total acid component comprises no less than about 35 mole % saturated aliphatic acids and no more than about 65 mole % unsaturated fatty acids, said total acid component being further characterized as comprising:

(a) more than about 20 mole % of mono-unsaturated fatty acids;
(b) less than about 15 mole % of poly-unsaturated fatty acids;
(c) more than about 20 mole % saturated aliphatic acids having 6 to 16 carbon atoms, including more than about 10 mole % saturated aliphatic acids having 6 to 14 carbon atoms; and
(d) less than about 15 mole % saturated aliphatic acids having 18 or more carbon atoms; and

(2) sulfurizing the transesterified triglyceride product mixture of step 1 with elemental sulfur to incorporate bound sulfur in an amount within the range from about 4 wt. % to about 14 wt. % based upon the transesterified triglyceride product mixture; and wherein the method optionally includes, before step (2) blending said transesterification reaction product with a compound selected from the group consisting of triglycerides, esterified fatty acids, α-olefins and mixtures thereof.

8. A method according to claim 7, additionally comprising the step of esterifying free acids in the transesterification reaction product with an alcohol component prior to the sulfurization step, the alcohol component for esterification preferably comprising one or more branched chain aliphatic primary alcohols.

9. A method according to claim 7 or 8, wherein said transesterification step is carried out in the presence of a transesterification catalyst, preferably tetrabutyl titanate, zinc acetate, sodium carbonate, sodium sulfate, stannous oxalate, p-toluenesulfonic acid, methanesulfonic acid, sulfuric acid, butylchlorotin dihydroxide, or phosphoric acid.

10. A method according to claim 9, wherein said transesterification catalyst is present in an amount within the range from about 0.01 to about 1 wt. %, preferably 0.03 to about 0.5 wt. %, based on the triglycerides.

11. A lubricating oil composition, comprising:
(1) a refined base oil, having lubricating oil viscosity and volatility properties; and
(2) a minor amount of a sulfurized triglyceride additive composition as claimed in any of claims 1 to 6.

12. A lubricating oil composition according to claim 11, wherein the sulfurized triglyceride additive composition is present in an amount within the range from about 0.05 to about 15 wt. %, preferably about 0.5 to about 5 wt. %, of the lubricating oil composition.

13. A fuel composition, comprising:
(a) a blended base fuel, having suitable volatility and combustion properties; and
(2) a minor amount of a sulfurized triglyceride additive composition as claimed in any of claims 1 to 6.

14. A composition according to claim 13, wherein the blended base fuel is a gasoline fuel, e.g. a diesel fuel, for use in a spark-ignition internal combustion engine, or is a heating oil, for use in an oil-fired burner assembly.

15. A composition according to claim 13 or 14, wherein the sulfurized triglyceride additive composition is present in an amount within the range from about 0.0005 to about 0.1 wt % of the fuel composition, preferably from about 0.0015 to about 0.05 wt. %, of the fuel composition.

**Patentansprüche**

1. Eine sulfurierte Triglycerid-Additivzusammensetzung, umfassend ein sulfuriertes, umgeestertes Triglycerid, worin die Gesamtsäurekomponente des Triglycerids nicht weniger als etwa 35 Mol-%, vorzugsweise mehr als 50 Mol-% gesättigte, aliphatische Säuren und nicht mehr als 65 Mol-% gesättigte Fettsäuren umfaßt, wobei die Gesamtsäurekomponente weiter dadurch gekennzeichnet ist, daß sie umfaßt:

a) mehr als etwa 20 Mol-%, vorzugsweise mehr als 35 Mol-% monoungesättigte Säuren;
b) weniger als etwa 15 Mol-%, vorzugweise weniger als 10 Mol-% polyungesättigte Fettsäuren;

c) mehr als etwa 20 Mol-% gesättigte, aliphatische Säuren mit 6 bis 16 Kohlenstoffatomen, einschließlich mehr als etwa 10 Mol-% gesättigte, aliphatische Säuren mit 6 bis 14 Kohlenstoffatomen; und

d) weniger als etwa 15 Mol-% gesättigte, aliphatische Säuren mit 18 oder mehr Kohlenstoffatomen.

2. Zusammensetzung nach Anspruch 1, worin die gesättigten Säuren mit 6 bis 14 Kohlenstoffatomen in einer Menge größer als etwa 15 Mol-%, vorzugsweise größer als etwa 35 Mol-%, vorliegen.

3. Zusammensetzung nach Anspruch 1 oder 2, welche zusätzlich ein Solubilisationmittel umfaßt.

4. Zusammensetzung nach Anspruch 3, worin das Solubilisationsmittel ein Triglycerid, ein Olefin, vorzugsweise enthaltend 8 bis 20 Kohlenstoffatome, ein Ester eine ungesättigten Carbonsäure oder eine Mischung aus zwei oder mehreren davon ist.

5. Zusammensetzung nach Anspruch 3 oder 4, worin das Solubilisationsmittel in einer Menge innerhalb des Bereichs von etwa 5 Gew.-% bis etwa 70 Gew.-%, vorzugsweise 5 Gew.-% bis etwa 55 Gew.-%, bezogen auf das umgeesterte Triglycerid, vorliegt.

6. Zusammensetzung nach einem der Ansprüche 1 bis 5, worin gebundener Schwefel in einer Menge innerhalb des Bereichs von etwa 4 Gew.-% bis etwa 14 Gew.-%, bezogen auf das Gesamtprodukt, vorliegt.

7. Verfahren zur Herstellung einer sulfurierten, umgeesterten Triglycerid-Additivzusammensetzung gemäß Anspruch 6, umfassend die Stufen:

(1) Umestern eines oder mehrerer Triglyceride mit einer oder mehreren organischen Säuren oder Estern davon, um ein umgeestertes Triglycerid zu ergeben, worin die Gesamtsäurekomponente nicht weniger als etwa 35 Mol-% gesättigte, aliphatische Säuren und nicht mehr als etwa 65 Mol-% ungesättigte Fettsäuren umfaßt, wobei die Gesamtsäurekomponente weiter dadurch gekennzeichnet ist, daß sie umfaßt

a) mehr als etwa 20 Mol-% monoungesättigte Fettsäuren,
b) weniger als etwa 15 Mol-% polyungesättigte Fettsäuren,
c) mehr als etwa 20 Mol-% gesättigte, aliphatische Säuren mit 6 bis 16 Kohlenstoffatomen, einschließlich mehr als etwa 10 Mol-% gesättigte, aliphatische Säuren mit 6 bis 14 Kohlenstoffatomen, und
d) weniger als etwa 15 Mol-% gesättigte, aliphatische Säuren mit 18 oder mehr Kohlenstoffatomen; und

(2) Sulfurieren der umgeesterten Triglyceridproduktmischung der Stufe (1) mit elementarem Schwefel, um gebundenen Schwefel in einer Menge innerhalb des Bereichs von etwa 4 Gew.-% bis etwa 14 Gew.-%, bezogen auf die umgeesterte Triglyceridproduktmischung, einzuarbeiten; und wobei das Verfahren gegebenenfalls vor der Stufe (2) das Mischen des Umesterungsreaktionsprodukts mit einer Verbindung, gewählt aus der Gruppe, bestehend aus Triglyceriden, veresterten Fettsäuren, α-Olefinen und Mischungen daraus, einschließt.

8. Verfahren nach Anspruch 7, welches zusätzlich die Stufe der Veresterung freier Säuren in dem umgeesterten Reaktionsprodukt mit einer Alkhoholkomponente vor der Sulfurierungsstufe umfaßt, wobei die Alkoholkomponente zur Veresterung vorzugsweise eine oder mehrere verzweigtkettige, aliphatische, primäre Alkohole umfaßt.

9. Verfahren nach Anspruch 7 oder 8, worin die Umesterungsstufe in Gegenwart eines Umesterungskatalysators, vorzugsweise Tetrabutyltitanat, Zinkacetat, Natriumcarbonat, Natriumsulfat, Stannooxalat, p-Toluolsulfonsäure, Methansulfonsäure, Schwefelsäure, Butylchlorzinndihydroxid oder Phosphorsäure, durchgeführt wird.

10. Verfahren nach Anspruch 9, worin der Umesterungskatalysator in einer Menge innerhalb des Bereichs von etwa 0,01 bis etwa 1 Gew.-%, vorzugsweise 0,03 bis etwa 0,5 Gew.-%, bezogen auf die Triglyceride, vorliegt.

11. Schmierölzusammensetzung, umfassend:

(1) ein raffiniertes Grundöl mit Schmierölviskositäts- und -flüchtigkeitseigenschaften; und

(2) eine geringere Menge einer sulfurierten Triglycerid-Additivzusammensetzung gemäß einem der Ansprüche 1 bis 6.

12. Schmierölzusammensetzung nach Anspruch 11, worin die sulfurierte Triglycerid-Additivzusammensetzung in einer Menge innerhalb des Bereichs von etwa 0,05 bis etwa 15 Gew.-%, vorzugsweise etwa 0,5 bis etwa 5 Gew.-% der Schmierölzusammensetzung vorliegt.

13. Brennstoffzusammensetzung, umfassend:

(1) einen gemischten Grundbrennstoff mit geeigneten Flüchtigkeits- und Verbrennungseigenschaften; und

(2) eine geringere Menge einer sulfurierten Triglycerid-Additivzusammensetzung gemäß einem der Ansprüche 1 bis 6.

14. Zusammensetzung nach Anspruch 13, worin der gemischte Grundbrennstoff ein Benzinbrennstoff, beispielsweise ein Dieselbrennstoff zur Verwendung in einem Otto-Zünder-Verbrennungsmotor oder ein Heizöl zur Verwendung in einem ölgefeuerten Brennersystem ist.

15. Zusammensetzung nach Anspruch 13 oder 14, worin die sulfurierte Triglycerid-Additivzusammensetzung in einer Menge innerhalb des Bereichs von etwa 0,0005 bis etwa 0,1 Gew.-% der Brennstoffzusammensetzung, vorzugsweise von etwa 0,0015 bis etwa 0,05 Gew.-% der Brennstoffzusammensetzung vorliegt.

**Revendications**

1. Composition sulfurisée d'additif à base de triglycérides, comprenant un triglycéride transestérifié sulfurisé, où le composant acide total du triglycéride comprend au moins environ 35 %-molaire, de préférence plus de 50 %-molaire, d'acides aliphatiques saturés, et au plus environ 65 %-molaire d'acides gras saturés, ledit composant acide total étant en outre caractérisé en ce qu'il comprend:

a) plus d'environ 20 %-molaire, de préférence plus de 35 %-molaire, d'acides monoinsaturés;

b) moins d'environ 15 %-molaire, de préférence moins de 10 %-molaire, d'acides gras polyinsaturés;

c) plus d'environ 20 %-molaire d'acides aliphatiques saturés ayant 6 à 14 atomes de carbone, y compris plus d'environ 10 %-molaire d'acides aliphatiques saturés ayant 6 à 15 atomes de carbone; et

d) moins d'environ 15 %-molaire d'acides aliphatiques saturés ayant 18 atomes de carbone, ou plus.

2. Composition selon la revendication 1, dans laquelle lesdits acides saturés ayant 6 à 14 atomes de carbone sont présents en une quantité supérieure à environ 15 %-molaire, de préférence supérieure à environ 35 %-molaire.

3. Composition selon la revendication 1 ou 2, qui comprend en outre un agent solubilisateur.

4. Composition selon la revendication 3, dans laquelle l'agent solubilisateur est un triglycéride, une oléfine contenant de préférence de 8 à 20 atomes de carbone, un ester d'un acide carboxylique insaturé, ou tout mélange d'au moins deux de ces substances.

5. Composition selon la revendication 3 ou 4, dans laquelle l'agent solubilisateur est présent en une quantité comprise dans l'intervalle allant d'environ 5% en poids à environ 70% en poids, de préférence de 5% en poids à environ 55% en poids, sur la base du triglycéride transestérifié.

6. Composition selon l'une quelconque des revendications 1 à 5, dans laquelle le soufre lié est présent en une quantité comprise dans l'intervalle allant d'environ 4% en poids à environ 14% en poids, sur la base du produit total.

7. Procédé pour la préparation d'une composition d'additif à base de triglycérides transestérifiés sulfurisés selon la revendication 6, comprenant les étapes consistant

(1) à transestérifier un ou plusieurs triglycérides avec un ou plusieurs acides organiques ou leurs esters, pour obtenir un triglycéride transestérifié dans lequel le composant acide total comprend au moins environ 35 %-molaire d'acides aliphatiques saturés, et au plus environ 65 %-molaire d'acides gras insaturés, ledit composant acide total étant en outre caractérisé en ce qu'il comprend

(a) plus d'environ 20 %-molaire d'acides gras monoinsaturés;
(b) moins d'environ 15 %-molaire d'acides gras polyinsaturés;
(c) plus d'environ 20 %-molaire d'acides aliphatiques saturés ayant 6 à 16 atomes de carbone, y compris plus d'environ 10 %-molaire d'acides aliphatiques saturés ayant 6 à 14 atomes de carbone; et
(d) moins d'environ 15 %-molaire d'acides aliphatiques saturés ayant 18 atomes de carbone, ou plus; et

(2) à sulfuriser le mélange de produits triglycérides transestérifiés de l'étape 1 avec du soufre élémentaire pour incorporer du soufre lié en une quantité comprise dans l'intervalle allant d'environ 4% en poids à environ 14% en poids, sur la base du mélange de produits triglycérides transestérifiés;

et où le procédé consiste, facultativement, avant l'étape (2), à mélanger ledit produit de réaction de transestérification à un composé choisi dans le groupe comprenant les triglycérides, les acides gras estérifiés, les α-oléfines et leurs mélanges.

8. Procédé selon la revendication 7, comprenant en outre l'étape consistant à estérifier les acides libres dans le produit de la réaction de transestérification avec un composant alcool avant l'étape de sulfurisation, le composant alcool destiné à l'estérification comprenant de préférence un ou plusieurs alcools primaires aliphatiques à chaîne ramifiée.

9. Procédé selon la revendication 7 ou 8, dans lequel ladite étape de transestérification est réalisée en présence d'un catalyseur de transestérification, de préférence le titanate de tétrabutyle, l'acétate de zinc, le carbonate de sodium, le sulfate de sodium, l'oxalate stanneux, l'acide p-toluènesulfonique, l'acide méthanesulfonique, l'acide sulfurique, le dihydroxyde de butylchlorétain, ou l'acide phosphorique.

10. Procédé selon la revendication 9, dans lequel ledit catalyseur de transestérification est présent en une quantité comprise dans l'intervalle allant d'environ 0,01 à environ 1% en poids, de préférence de 0,03 à environ 0,5% en poids, sur la base des triglycérides.

11. Composition d'huile lubrifiante, comprenant:

(1) une huile de base raffinée, présentant des propriétés de viscosité et de volatilité des huiles lubrifiantes; et

(2) une quantité mineure d'une composition d'additif à base de triglycérides sulfurisés selon l'une quelconque des revendications 1 à 6.

12. Composition d'huile lubrifiante selon la revendication 11, dans laquelle la composition d'additif à base de triglycérides sulfurisés est présente en une quantité comprise dans l'intervalle allant d'environ 0,05 à environ 15% en poids, de préférence d'environ 0,5 à environ 5% en poids de la composition d'huile lubrifiante.

13. Composition de combustible, comprenant:

(1) un combustible de base mélangé, présentant des propriétés convenables de volatilité et de combustion; et

(2) une quantité mineure d'une composition d'additifs à base de triglycérides sulfurisés selon l'une quelconque des revendications 1 à 6.

14. Composition selon la revendication 13, dans laquelle le combustible de base mélangée est un carburant essence, par exemple un combustible diesel, pour utilisation dans un moteur à combustion interne à allumage par étincelle, ou bien est un fuel oil domestique, pour utilisation dans un ensemble à brûleurs chauffé au fuel.

15. Composition selon la revendication 13 ou 14, dans laquelle la composition d'additif à base de triglycérides sulfurisés est présente en une quantité comprise dans l'intervalle allant d'environ 0,0005 à environ 0,1% en poids de la composition de combustible, de préférence d'environ 0,0015 à environ 0,05% en poids de la composition de combustible.